# EUROPEAN PATENT APPLICATION

(11) **EP 3 545 996 A1**
(43) Date of publication of application: **02.10.2019**
(21) Application number: 18305346.1
(22) Date of filing: 28.03.2018
(51) Int. Cl.: A61M 5/315

(54) **INJECTION DEVICE WITH DISTANCE SENSOR**

(71) Applicant: Sanofi, 75008 Paris (FR)
(72) Inventor: The designation of the inventor has not yet been filed
(74) Representative: Sanofi Aventis Deutschland GmbH

(57) **Abstract**

The present invention refers to an injection device comprising a housing (5) with a receptacle (2) for the product, a dosing mechanism for setting a product dosage to be administered and for displaying the set product dosage and a dispensing mechanism for dispensing the product. The dispensing mechanism may comprise a piston rod (8), which is moveable relative to the housing (5) in a dispensing direction in order to eject the set product dosage in a dispensing stroke corresponding to the set product dosage, and a spring (7b), which is arranged such that the length of the spring (7b) changes during ejecting the set product dosage. The device further comprises a first detector (21) for detecting a change in the length of the spring (7b) and a feedback generator mechanism (20) connected to the first detector (21) for generating a feedback responsive to signals received from the first detector (21).

## Description

The present invention is generally directed to an injection device, i.e. a drug delivery device for selecting and dispensing a number of user variable doses of a medicament.

Pen type drug delivery devices have application where regular injection by persons without formal medical training occurs. This may be increasingly common among patients having diabetes where self-treatment enables such patients to conduct effective management of their disease. In practice, such a drug delivery device allows a user to individually select and dispense a number of user variable doses of a medicament. The present invention is not directed to so called fixed dose devices which only allow dispensing of a predefined dose without the possibility to increase or decrease the set dose.

There are basically two types of drug delivery devices: resettable devices (i.e., reusable) and non-resettable (i.e., disposable). For example, disposable pen delivery devices are supplied as self-contained devices. Such self-contained devices do not have removable pre-filled cartridges. Rather, the pre-filled cartridges may not be removed and replaced from these devices without destroying the device itself. Consequently, such disposable devices need not have a resettable dose setting mechanism. The present invention is applicable for both types of devices, i.e. for disposable devices as well as for reusable devices.

These types of pen delivery devices (so named because they often resemble an enlarged fountain pen) generally comprise three primary elements: a cartridge section that includes a cartridge often contained within a housing or holder; a needle assembly connected to one end of the cartridge section; and a dosing section connected to the other end of the cartridge section. A cartridge (often referred to as an ampoule) typically includes a reservoir that is filled with a medication (e.g., insulin), a movable rubber type bung or stopper located at one end of the cartridge reservoir, and a top having a pierceable rubber seal located at the other, often necked-down, end. A crimped annular metal band is typically used to hold the rubber seal in place. While the cartridge housing may be typically made of plastic, cartridge reservoirs have historically been made of glass.

The needle assembly is typically a replaceable double-ended needle assembly. Before an injection, a replaceable double-ended needle assembly is attached to one end of the cartridge assembly, a dose is set, and then the set dose is administered. Such removable needle assemblies may be threaded onto, or pushed (i.e., snapped) onto the pierceable seal end of the cartridge assembly.

The dosing section or dose setting mechanism is typically the portion of the pen device that is used to set (select) a dose. During an injection, a spindle or piston rod contained within the dose setting mechanism presses against the bung or stopper of the cartridge. This force causes the medication contained within the cartridge to be injected through an attached needle assembly. After an injection, as generally recommended by most drug delivery device and/or needle assembly manufacturers and suppliers, the needle assembly is removed and discarded.

A further differentiation of drug delivery device types refers to the drive mechanism: There are devices which are manually driven, e.g. by a user applying a force to an injection button, devices which are driven by a spring or the like and devices which combine these two concepts, i.e. spring assisted devices which still require a user to exert an injection force. The spring-type devices involve springs which are preloaded and springs which are loaded by the user during dose selecting. Some stored-energy devices use a combination of spring preload and additional energy provided by the user, for example during dose setting.

An injection device comprising a housing with a receptacle for the product, a dosing mechanism for setting a product dosage to be administered and for displaying the set product dosage and a dispensing mechanism for dispensing the product is known e.g. from EP 2 814 547 B1 which discloses a manually driven device or from WO 2014/117944 A1, WO 2016/016184 A1 or WO 2017/134131 A1 which disclose spring driven devices.

While some of these known devices are provided with a feedback mechanism for generating a feedback suitable for indicating the action of dose dispensing by providing a non-visual feedback signal to a user, WO 2016/001304 A1 discloses an injection device with a feedback mechanism which is provided with a clicker arrangement activated only at the end of dose dispensing, thereby providing an additional audible feedback in the form of a 'click', distinct from the 'clicks' provided during dispense, to inform the user that the device has returned to its zero position. This distinct feedback is only created at the end of dose delivery.

It is an object of the present invention to provide an improved alternative to the above solutions. Especially, it is an object of the present invention to provide an injection device or a drug delivery device giving a reliable feedback to users at the beginning and/or end of the dispensing process. Preferably, the mechanism does not generate a signal during dose resetting.

This object is solved by an injection device according to claim 1.

An injection device according to the present invention comprises a housing with a receptacle for the product, a dosing mechanism for setting a product dosage to be administered and for displaying the set product dosage and a dispensing mechanism for dispensing the product. The dispensing mechanism of the device may comprise a piston rod, which is movable relative to the housing in a dispensing direction in order to eject the set product dosage in a dispensing stroke corresponding to the set product dosage, and a spring, which is arranged such that the length of the spring changes during ejecting the set product dosage. The present invention is based on the idea that detecting movements of component parts of the injection device may be used to identify the beginning and/or the end of dose dispensing and that a feedback signal may be generated based on this information. For example, the device further may comprise a first detector for detecting a change in the length of the spring and a feedback generator mechanism connected to the first detector for generating a feedback responsive to signals received from the first detector. In addition or as an alternative, the device may comprise at least two component parts, which are axially movable relative to each other during ejecting the set product dosage, and a second detector for detecting the position of the first component part relative to the second component part, wherein the feedback generator mechanism is connected to the second detector for generating a feedback responsive to signals received from the second detector. The at least two component parts may be sleeves which are rotationally constrained to each other.

The idea of detecting movements of component parts of the injection device to identify the beginning and/or the end of dose dispensing and generating a feedback signal based on this information is applicable to a variety of different injection devices irrespective of the different concepts of the devices, i.e. the invention finds application in re-usable and disposable devices, in manually driven devices and in fully or partially spring driven devices. Different concepts of dosing mechanisms or dispensing mechanisms may be applied including devices requiring a rotation of a component part or requiring an axial movement of a component part for dose setting and/or devices with a rotating piston rod, e.g. along a helical path, or devices with a piston rod which is rotationally constrained to the housing.

According to an embodiment of the invention, the spring is interposed between a component part axially constrained to the housing and a further component part axially movable relative to the housing. In more detail, the further component part may be a toothed ring axially movable relative to corresponding teeth of the housing against the bias of the spring. For example, the toothed ring and the corresponding teeth of the housing form a ratchet permitting rotation of the toothed ring relative to the housing in one direction while blocking relative rotation in the opposite direction. Preferably, rotation of the toothed ring relative to the housing is permitted only during dose dispensing. This rotation may require that teeth of the toothed ring slip or jump over the corresponding teeth of the housing against the bias of the spring which urges the teeth into engagement.

The first detector may comprise a sensor adapted to detect the change in the length of the spring by detecting a change in the electrical resistance of the spring caused by the change in the length of the spring and/or an electrical field generated by the spring caused by the change in the length of the spring. For example, an induction loop may be used for detecting this change of the spring. As an alternative, the injection device may comprising a magnetic component part located such that the change in the length of the spring causes a change in of the magnetic field of the magnetic component part, wherein the first detector comprises a sensor adapted to detect the change in the length of the spring by detecting the change of the magnetic field of the magnetic component part caused by the change in the length of the spring.

If the injection device comprising the at least two, e.g. sleeve-like, component parts, which are axially movable relative to each other during ejecting the set product dosage the second detector for detecting the relative position of one of these component parts with respect to the other may comprise at least one of an optical sensor, a capacitive sensor, an inductive sensor, a magnetic sensor, a galvanic sensor or switch and a mechanical sensor.

The feedback generator mechanism is preferably adapted and arranged such that a start of dispensing feedback is generated upon detection of the beginning of a change in the length of the spring by means of the first detector. In addition or as an alternative, the feedback generator mechanism may be adapted and arranged such that an end of dispensing feedback is generated upon detection the end of a change in the length of the spring by means of the first detector.

The feedback generator may be provided within the housing of the injection device. For example, the feedback generator and the first and second detector may be permanently provided within the housing of the injection device. This includes embodiments in which the feedback generator is connected to at least one of the first and second detector by wire. As an alternative, the feedback generator mechanism may be detachable from the housing and/or may be connected to at least one of the first and second detector by means of a wireless connection. In other words, the feedback generator may be an add-on device which may be attached to the housing and removed from the housing.

The feedback generator may be adapted to generate the feedback signal immediately when receiving the information from one of the detectors that dose dispensing is stopped, i.e. by detecting that the button or clutch is in the dose setting position and/ that the at least one sleeve does not rotate. As an alternative, generation of a feedback signal may be delayed for a pre-set time, e.g. a dwelling time for which the user is typically asked to wait until the needle may be retracted from the skin. As a further alternative, an additional, e.g. different, feedback signal may be generated at the end of the dwelling time.

Further, if the detectors are adapted and arranged for detecting the amount of the set dosage, the feedback generator may be adapted to generate a feedback signal only if the set dose has been fully dispensed and/or may generate a different signal if the set dose has not been fully dispensed.

The injection device typically comprises a cartridge containing a medicament. The term "medicament", as used herein, means a pharmaceutical formulation containing at least one pharmaceutically active compound,
wherein in one embodiment the pharmaceutically active compound has a molecular weight up to 1500 Da and/or is a peptide, a proteine, a polysaccharide, a vaccine, a DNA, a RNA, an enzyme, an antibody or a fragment thereof, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exendin-3 or exendin-4 or an analogue or derivative of exendin-3 or exendin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

Exendin-4 for example means Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
H-(Lys)5-des Pro36, des Pro37 Exendin-4(1-39)-NH2,
des Pro36 Exendin-4(1-39),
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(O2)25, Asp28] Exendin-4(1-39),
des Pro36 [Met(O)14 Trp(O2)25, IsoAsp28] Exendin-4(1-39); or
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(O2)25, Asp28] Exendin-4(1-39),
des Pro36 [Met(O)14 Trp(O2)25, IsoAsp28] Exendin-4(1-39),
wherein the group -Lys6-NH2 may be bound to the C-terminus of the Exendin-4 derivative;

or an Exendin-4 derivative of the sequence
des Pro36 Exendin-4(1-39)-Lys6-NH2 (AVE0010),
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2,
H-Asn-(Glu)5des Pro36, Pro37, Pro38 [Asp28] Exendin-4(1-39)-NH2,
des Pro36, Pro37, Pro38 [Asp28] Exendin-4(1-39)-(Lys)6-NH2,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(O2)25, Asp28] Exendin-4(1-39)-Lys6-NH2,
H-des Asp28 Pro36, Pro37, Pro38 [Trp(O2)25] Exendin-4(1-39)-NH2,
H-(Lys)6-des Pro36, Pro37, Pro38 [Trp(O2)25, Asp28] Exendin-4(1-39)-NH2,
H-Asn-(Glu)5-des Pro36, Pro37, Pro38 [Trp(O2)25, Asp28] Exendin-4(1-39)-NH2,
des Pro36, Pro37, Pro38 [Trp(O2)25, Asp28] Exendin-4(1-39)-(Lys)6-NH2,
H-(Lys)6-des Pro36, Pro37, Pro38 [Trp(O2)25, Asp28] Exendin-4(1-39)-(Lys)6-NH2,
H-Asn-(Glu)5-des Pro36, Pro37, Pro38 [Trp(O2)25, Asp28] Exendin-4(1-39)-(Lys)6-NH2,
H-(Lys)6-des Pro36 [Met(O)14, Asp28] Exendin-4(1-39)-Lys6-NH2,
des Met(O)14 Asp28 Pro36, Pro37, Pro38 Exendin-4(1-39)-NH2,
H-(Lys)6-desPro36, Pro37, Pro38 [Met(O)14, Asp28] Exendin-4(1-39)-NH2,
H-Asn-(Glu)5-des Pro36, Pro37, Pro38 [Met(O)14, Asp28] Exendin-4(1-39)-NH2,
des Pro36, Pro37, Pro38 [Met(O)14, Asp28] Exendin-4(1-39)-(Lys)6-NH2,
H-(Lys)6-des Pro36, Pro37, Pro38 [Met(O)14, Asp28] Exendin-4(1-39)-(Lys)6-NH2,
H-Asn-(Glu)5 des Pro36, Pro37, Pro38 [Met(O)14, Asp28] Exendin-4(1-39)-(Lys)6-NH2,
H-Lys6-des Pro36 [Met(O)14, Trp(O2)25, Asp28] Exendin-4(1-39)-Lys6-NH2,
H-des Asp28 Pro36, Pro37, Pro38 [Met(O)14, Trp(O2)25] Exendin-4(1-39)-NH2,
H-(Lys)6-des Pro36, Pro37, Pro38 [Met(O)14, Asp28] Exendin-4(1-39)-NH2,
H-Asn-(Glu)5-des Pro36, Pro37, Pro38 [Met(O)14, Trp(O2)25, Asp28] Exendin-4(1-39)-NH2,
des Pro36, Pro37, Pro38 [Met(O)14, Trp(O2)25, Asp28] Exendin-4(1-39)-(Lys)6-NH2,
H-(Lys)6-des Pro36, Pro37, Pro38 [Met(O)14, Trp(O2)25, Asp28] Exendin-4(S1-39)-(Lys)6-NH2,
H-Asn-(Glu)5-des Pro36, Pro37, Pro38 [Met(O)14, Trp(O2)25, Asp28] Exendin-4(1-39)-(Lys)6-NH2;
or a pharmaceutically acceptable salt or solvate of any one of the afore-mentioned Exendin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Antibodies are globular plasma proteins (-150 kDa) that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two β sheets create a "sandwich" shape, held together by interactions between conserved cysteines and other charged amino acids.

There are five types of mammalian Ig heavy chain denoted by α, δ, ε, γ, and µ. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (CH) and the variable region (VH). In one species, the constant region is essentially identical in all antibodies of the same isotype, but differs in antibodies of different isotypes. Heavy chains γ, α and δ have a constant region composed of three tandem Ig domains, and a hinge region for added flexibility; heavy chains µ and ε have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single Ig domain.

In mammals, there are two types of immunoglobulin light chain denoted by λ and κ. A light chain has two successive domains: one constant domain (CL) and one variable domain (VL). The approximate length of a light chain is 211 to 217 amino acids. Each antibody contains two light chains that are always identical; only one type of light chain, κ or λ, is present per antibody in mammals.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

An "antibody fragment" contains at least one antigen binding fragment as defined above, and exhibits essentially the same function and specificity as the complete antibody of which the fragment is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

Pharmaceutically acceptable salts are for example acid addition salts and basic salts. Acid addition salts are e.g. HCI or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

Non-limiting, exemplary embodiments of the invention will now be described with reference to the accompanying drawings, in which:
- Figure 1: shows an exploded view of the individual parts of an embodiment of an injection device according to the invention; and
- Figure 2: shows a sectional view of a detail of the embodiment of Figure 1.

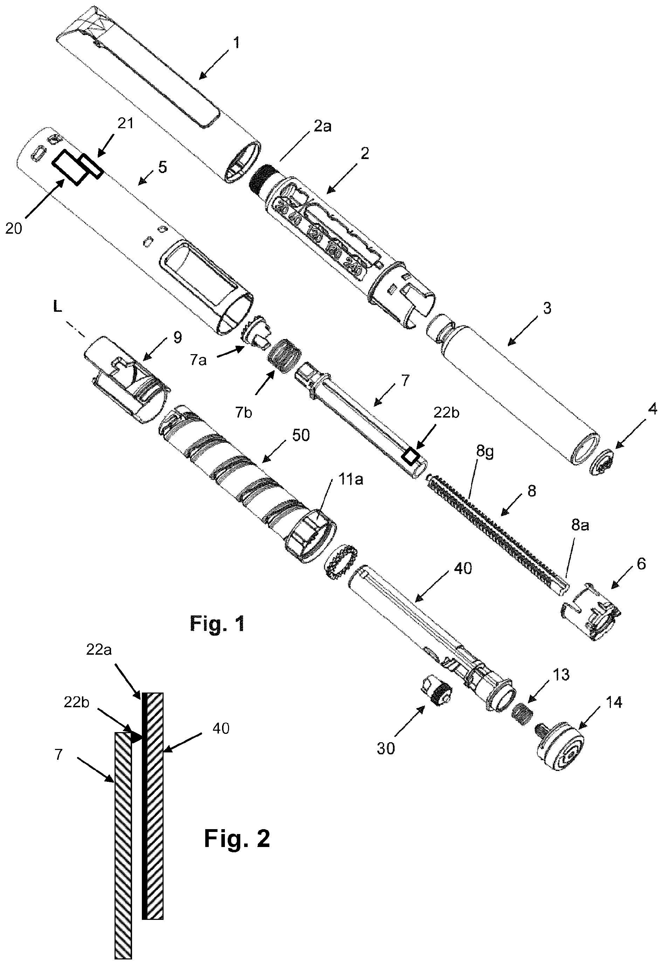

Figure 1 shows an exploded view of the individual parts of an embodiment of an injection device according to the invention. This embodiment is designed as a so-called single-use pen. That is to say, the injection device is issued to the user fully assembled, i.e., with product to be administered.

The typical course of the injection process may be as follows: the user removes the protective cap 1 from the injection device and mounts an injection needle (not shown) on the needle holder 2a of a receptacle 2. Now the dosage can be adjusted via the rotary knob 11a.

For this purpose, the rotary knob 11a is turned so that the dosing sleeve 50, which forms a dosing mechanism or is part of a dosing mechanism, is screwed out of the injection device. The dosing sleeve 50 is screwed out of the injection device until the desired dosage is displayed in the window of the threaded sleeve 9. If an excessively high dosage is inadvertently set, the dosage can be corrected by turning the rotary knob in the opposite direction, whereby the dosing sleeve 50 is screwed back into the housing. The dosing device limits the maximum adjustable dosage to a predetermined value. If there is an attempt to screw the dosing sleeve out of the housing past this value, a radial limit stop on the dosing sleeve 50 and a counter-limit stop on the threaded sleeve 9 prevent further rotation by mutual interaction. During the dosing and correction movements, the dosing sleeve 50 rotates relative to the coupling sleeve 40. The coupling sleeve 40 is held rotationally fixedly in a form fit or friction fit against the housing 5 by a reverse rotation lock, for example by means of a toothed ring 7a rotationally fixed to the threaded nut 7 and biased axially towards a corresponding set of ratchet teeth on an inner surface of the housing 5 by means of a spring 7b. This ratchet between the housing 5 and the threaded nut 7 may form a clicker generating an audible and/or tactile feedback during dose dispensing as the teeth of ring 7a slip over the corresponding teeth of housing 5.

The coupling sleeve 40 is permanently rotationally locked to the axially stationary threaded nut 7 by splines permitting relative axial movement between the coupling sleeve 40 which is axially entrained by the dosing sleeve 50 and the stationary threaded nut 7. The threaded nut 7 may be retained in the housing 5 by means of an insert 6 holding the threaded nut 7 against the bias of spring 7b. A toothed ring is shown in Figure 1 which is interposed between rotary knob 11a and a flange on coupling sleeve 40 such that relative rotation of the dosing sleeve 50 and the coupling sleeve 40 is permitted during dose setting.

If the desired dosage has been set, the injection needle can be inserted at the intended position on the body of the user. Then the user pushes the ejection button 14 in the distal axial direction against the bias of spring 13 which is interposed between button 14 and coupling sleeve 40. This axial movement blocks a relative rotation between the coupling sleeve 40 and the dosing sleeve 50 by means of the toothed ring. In case of further pressure in the distal axial direction, the dosing sleeve begins to move back into the housing in a screwing motion. Because of the established rotational lock between the dosing sleeve 50 and the coupling sleeve 40, the coupling sleeve 40 carries out the same movement as the dosing sleeve 50. Because the coupling sleeve 40 is permanently rotationally locked to the axially stationary threaded nut 7, the rotational movement of the dosing sleeve 50 is transmitted to the threaded nut 7. No axial forces are transmitted to the threaded nut 7, because the coupling sleeve 40 is mounted axially movably on the threaded nut 7. Thus, the rotating threaded nut 7 produces an axial movement of the threaded piston rod 8 in the distal direction, wherein the latter is guided axially and locked rotationally in the housing 5 by means of splines 8a and is in threaded engagement with threaded nut 7 by means of an external thread 8g. The flange 4 acts on the plug of the cartridge 3 and pushes it, corresponding to the displacement of the threaded piston rod 8 in the distal direction as well, wherein the previously set dosage can be ejected or administered. At the end of the administration, when the dosing sleeve has been completely screwed back into the housing, radial stops on the dosing sleeve 50 and the threaded sleeve 9 prevent further ejection and overrotation of the dosing device.

The embodiment of Figure 1 shows an optional limiting device which ensures that the most recently set dosage can be completely ejected or injected. For this purpose, the dosing sleeve 50 has a coaxially applied inner toothing and the coupling sleeve 40 has a lateral cutout in which the stop wheel 30 is inserted. The function of the limiting device is described in EP 2 814 547 B1 in more detail.

The embodiment of Figure 1 further shows a feedback generator mechanism 20 connected to a first detector 21 and a second detector 22 for generating a feedback responsive to signals received from the first and second detectors 21, 22. The feedback generator mechanism 20 is depicted as a component part being disposed on the outer surface of the housing 5. It may be permanently attached to the housing 5 or may be an integral part thereof or may be a separate, detachable unit. The feedback generator mechanism 20 may comprise a power supply and a PCB suitable for generating an audible and/or tactile and/or visual feedback, e.g. a sound and/or a vibration and/or a light.

The first detector 21 is disposed in the housing 5 at a position suitable for detecting changes in the length of the spring 7a. The first detector 21 may be integrated in the feedback generator mechanism 20 which is positioned on or in the housing 5. Detection of a change in the length of spring 7a by the first detector 21 is effected by detecting a change of the electrical field. For example, an induction loop may be used for this detection.

Figure 2 shows in a sectional view a portion of the coupling sleeve 40 and a portion of the threaded nut 7. As mentioned above, the coupling sleeve 40 and the threaded nut 7 are splined together such that a relative axial movement during dose dispensing is permitted. This relative axial movement during dose dispensing may be used to identify whether the device is actually dispensing a dose and/or to detect beginning or stopping of this dose dispensing operation. For example, in a device as shown in Figures 1 and 2 this relative axial movement of the coupling sleeve 40 and the threaded nut 7 occurs only during dose dispensing if correcting a too high dose is not permitted. If dose correction is permitted, this relative axial movement of the coupling sleeve 40 and the threaded nut 7 may be used together with additional information to identify whether the device is actually dispensing a dose and/or to detect beginning or stopping of this dose dispensing operation. Such additional information may include e.g. detecting changes in the length of the spring 7a, detecting whether rotation of the toothed ring 7b occurs and/or detecting the position of the button 14 relative to the housing 5 or other component parts.

A second detector 22 is shown in Figure 2 interposed between the coupling sleeve 40 and the threaded nut 7. In more detail, the second detector 22 may comprise at least one, e.g. axially extending, electrically conductive portion 22a provided e.g. on the coupling sleeve 40 and an electrically conductive contact portion 22b provided on the threaded nut 7 such that it abuts the electrically conductive portion 22a on the coupling sleeve 40. Relative axial displacement of the coupling sleeve 40 and the threaded nut 7 may be detected by measuring the electrical resistance between one end of the electrically conductive portion 22a and the contact portion 22b.

As an alternative, the coupling sleeve 40 may be provided with at least two electrically conductive portions 22a provided at axially spaced positions on the coupling sleeve 40 such that the contact portion 22b abuts a respective electrically conductive portion 22a only if the coupling sleeve 40 and the threaded nut 7 are in predefined relative axial positions, e.g. a dose dispensing start position and a dose dispensing end position. In other words, the second detector 22 may be used like a switch providing signals indicative of certain positions of the coupling sleeve 40 relative to the (e.g. axially constrained) threaded nut 7.

If the feedback generator mechanism 20 uses the signals of the first detector 21 and the second detector 22, a malfunction of the device will not result in generating the respective feedback signals. For example, if the needle would be blocked such that dispensing is prevented, the length of spring 7a would not change although threaded nut 7 and the coupling sleeve 40 may move axially relative to each other.

As mentioned above, the feature of a feedback generator mechanism 20 using the signals of a first detector 21 and/or a second detector 22 for verifying that a device starts or stops dispensing a dose may be applied to other drug delivery devices having a spring-like component part changing its length during dose dispensing and/or component parts, e.g. sleeves, performing a relative movement, e.g. axial displacement, during dose dispensing. Examples for devices into which the feedback generator mechanism 20 with the first detector 21 and the second detector 22 may be implemented are disclosed in WO 2014/117944 A1, WO 2016/016184 A1, WO 2017/134131 A1 or in WO 2016/001304 A1.

### Reference Numerals

- 1: cap
- 2: receptacle
- 2a: needle holder
- 3: cartridge
- 4: flange
- 5: housing
- 6: insert
- 7: threaded nut
- 7a: toothed ring
- 7b: spring
- 8: piston rod
- 8a: spline
- 8g: thread
- 9: threaded sleeve
- 11a: rotary knob
- 13: spring
- 14: button
- 20: feedback generator mechanism
- 21: first detector
- 22: second detector
- 22a: electrically conductive portion
- 22b: contact portion 22b
- 30: stop wheel
- 40: coupling sleeve
- 46: web
- 50: dosing sleeve

## Claims

1. An injection device comprising a housing (5) with a receptacle (2) for the product, a dosing mechanism for setting a product dosage to be administered and for displaying the set product dosage and a dispensing mechanism for dispensing the product, the dispensing mechanism comprising a piston rod (8), which is moveable relative to the housing (5) in a dispensing direction in order to eject the set product dosage in a dispensing stroke corresponding to the set product dosage, and a spring (7b), which is arranged such that the length of the spring (7b) changes during ejecting the set product dosage,
**characterized in that** the device further comprises a first detector (21) for detecting a change in the length of the spring (7b) and a feedback generator mechanism (20) connected to the first detector (21) for generating a feedback responsive to signals received from the first detector (21).

2. The injection device according to claim 1, wherein the spring (7b) is interposed between a component part (7) axially constrained to the housing (5) and a further component part (7a) axially movable relative to the housing (5).

3. The injection device according to claim 2, wherein the further component part (7a) is a toothed ring axially movable relative to corresponding teeth of the housing (5) against the bias of the spring (7b).

4. The injection device according to claim 3, wherein the toothed ring (7a) and the corresponding teeth of the housing (5) form a ratchet permitting rotation of the toothed ring (7a) relative to the housing (5) in one direction while blocking relative rotation in the opposite direction.

5. The injection device according to any one of the preceding claims, wherein the first detector (21) comprises a sensor adapted to detect the change in the length of the spring (7b) by detecting a change in the electrical resistance of the spring (7b) caused by the change in the length of the spring (7b) and/or an electrical field generated by the spring (7b) caused by the change in the length of the spring (7b).

6. The injection device according to any one of the preceding claims, further comprising a magnetic component part (40) located such that the change in the length of the spring (7b) causes a change in of the magnetic field of the magnetic component part (40), wherein the first detector (21) comprises a sensor adapted to detect the change in the length of the spring (7b) by detecting the change of the magnetic field of the magnetic component part (40) caused by the change in the length of the spring (7b).

7. The injection device according to any one of the preceding claims, further comprising at least two component parts (7; 40), which are axially movable relative to each other during ejecting the set product dosage, and a second detector (22) for detecting the position of the first component part (7) relative to the second component part (40), wherein the feedback generator mechanism (20) is connected to the second detector (22) for generating a feedback responsive to signals received from the second detector (22).

8. The injection device according to claim 7, wherein the at least two component parts (7; 40) are sleeves which are rotationally constrained to each other.

9. The injection device according to any one of claims 7 or 8, wherein the second detector (22) comprises at least one of an optical sensor, a capacitive sensor, an inductive sensor, a magnetic sensor, a galvanic sensor or switch and a mechanical sensor.

10. The injection device according to any one of the preceding claims, wherein the feedback generator mechanism (20) is adapted and arranged such that a start of dispensing feedback is generated upon detection of the beginning of a change in the length of the spring (7b) by means of the first detector (21).

11. The injection device according to any one of the preceding claims, wherein the feedback generator mechanism (20) is adapted and arranged such that an end of dispensing feedback is generated upon detection the end of a change in the length of the spring (7b) by means of the first detector (21).

12. The injection device according to any one of the preceding claims, wherein the feedback generator mechanism (20) is provided within the housing (5) and/or is connected to at least one of the first and second detector (21, 22) by wire.

13. The injection device according to any one of claims 1 to 13, wherein the feedback generator mechanism detachable from the housing (5) and/or is connected to at least one of the first and second detector (21, 22) by means of a wireless connection.

14. The injection device according to any one of the preceding claims further comprising a cartridge containing a medicament.
